# EUROPEAN PATENT APPLICATION

(11) **EP 4 692 043 A1**
(43) Date of publication of application: **11.02.2026**
(21) Application number: 24193351.4
(22) Date of filing: 07.08.2024
(51) Int. Cl.: C07C 209/32, C07C 209/38, C07C 209/40, C07C 211/51, C07C 211/52, C07C 213/02, C07C 215/80

(54) **USE OF A MECHANOCHEMICAL METHOD FOR SELECTIVELY REDUCING NITROGEN-CONTAINING COMPOUNDS**

(71) Applicant: Université de Montpellier, 34090 Montpellier (FR); Centre National de la Recherche Scientifique, 75016 Paris (FR); Ecole Nationale Supérieure de Chimie de Montpellier, 34296 Montpellier (FR)
(72) Inventor: COLACINO, Evelina, 34090 Montpellier (FR); BORDIER, Corentin, 72120 Saint-Calais (FR); SOLORZANO-RODRIGUEZ, Ruben, 34170 Castelnau-le-Lez, France (FR); CHATZIGIANNIS, Christos, 34090 Montpellier (FR)
(74) Representative: Cabinet Grosset-Fournier & Demachy

(57) **Abstract**

The invention relates to the use of a mechanochemical method for selectively reducing a compound containing at least one nitro, nitroso-, N-hydroxylamino group in liquid-assisted grinding (LAG) conditions into an amine, with a metal-free reactant composition comprising at least one reducing agent and at least one liquid hydrogen surrogate, the reactant composition being free from molecular H2 and being free from base,
said liquid-assisted grinding (LAG) conditions occuring in a device chosen among: planetary ball-mill, resonant acoustic mixing (RAM), screw extrusion (single, twin or multi), drum mill, dyno-mill, or vibrating eccentric mill.

## Description

The present invention refers to the use of a mechanochemical method for selectively reducing nitrogen-containing compounds and a process for reducing said compounds to their respective amine derivatives.

### Background of the Invention

Nitro-, nitroso-, N-hydroxylamino-compounds are important starting materials in organic synthesis, in which they are generally used as precursors to amine derivatives.

Traditional hydrogenation methods used in solution-based synthesis include a recyclable precious metal catalyst and as a hydrogen source, gaseous H₂. However, due to their toxicity and especially for application targeting the preparation of active pharmaceutical ingredients, their careful elimination (or reduction of their amount below the thresholds imposed by the pharmacopeia) requires careful and costly purification method, generating toxic waste that needed to be retreated thereof. Precious metals of the catalysts belong to the category of "critical raw material", because their main sources are located in areas of the world undergoing geopolitical issues that can make them difficult to be exploited or because their availability worldwide has diminished due to intensive exploitation of the monetary sites, leading to shortage of some critical raw materials.

Furthermore, a wide range of reducing agents are used for the catalytic transformation of nitro derivatives in particular nitroarenes to amines. Each of them possesses advantages and disadvantages with respect to their handling, cost and environmental impact as well as hazardousness. Industrial reduction methods for the production of bulk chemicals are commonly conducted using molecular hydrogen, which is atom-efficient and inexpensive. Commonly, direct catalytic hydrogenations of nitroarenes are conducted under relatively high pressure and temperatures and in the presence of metals which often which belong to the category of "critical raw material".

Another alternative gaseous reducing system is based on water gas shift reaction employing a combination of CO and water. Despite the lower price of CO compared to H₂, it is very toxic and requires special handling and safety equipment.

There is therefore great interest in identifying new methods for reducing nitrogen-containing compounds to amines, including the development of new sustainable methodologies that are also economical. In this context, attention is mainly focused on alternative reduction methods to hydrogenation, with non-toxic reagents, with low environmental impact and without metals, especially on the part of companies interested in the synthesis of organic molecules, including complex ones, which can be chiral or non-chiral, but are characterized by the presence of a plurality of functional groups, the manipulation of which certainly requires the use of highly chemoselective methodologies.

The reduction method proposed in this invention with mechanochemical methods in the absence of solvents or in substochiometric amounts, can bypass all these drawbacks that refer to standard solution chemistry protocols. To begin with, the reduction via cheap, easily available and non-toxic reducing agent includes green and safe chemicals, instead of metals and H₂ gas that do not harm the environment and with the use of mechanochemical methods where green solvents are used in absence or substochiometric amounts, if not at all, make the method of the present application sustainable in addition to economic and eco-friendly. The method is robust, fast, under mild conditions and includes an easily scalable set-up for industrial applications and it generates no harmful or toxic waste and by-product(s) (e.g., NaHSO₃). It can also be noted that the by-product generated are not harmful and not toxic.

### Brief summary of the invention

The Applicant has surprisingly found that a metal-free reactant composition comprising at least one reducing agent and at least one hydrogen surrogate can be used for the selective synthesis of amine by reduction of nitro-, nitroso-, N-hydroxylamino compounds by a mechanochemical method.

More particularly, one of the objects of the present invention is the use of a mechanochemical method for selectively reducing a compound containing at least one nitro, nitroso-, N-hydroxylamino group in liquid-assisted grinding (LAG) conditions by a device chosen among: planetary ball-mill, resonant acoustic mixing (RAM), screw extrusion (single, twin or multi), drum mill, dyno-mill, or vibrating eccentric mill, or in solvent-free conditions, with a metal-free reactant composition comprising at least one reducing agent and at least one hydrogen surrogate.

Another object of the present invention is a mechanochemical process for selectively reducing a compound containing at least one nitro, nitroso, N-hydroxylamino group to an amine derivative by reacting at least one reducing agent with at least one hydrogen surrogate and in absence of any catalyst metal, in absence of gas H₂ and in absence of base, in liquid-assisted grinding (LAG) conditions or in solvent-free conditions.

### Detailed description of the invention

In the context of the present invention, the reactant composition may comprise at least one hydrogen surrogate.

### Hydrogen surrogate

As used herein, the term "hydrogen surrogate" refers to a substitute of hydrogen and/or a proton source.

The invention concerns the use of a mechanochemical method for selectively reducing a compound containing at least one nitro, nitroso-, N-hydroxylamino group in liquid-assisted grinding (LAG) conditions into an amine or in solvent-free conditions, with a metal-free reactant composition comprising at least one reducing agent and at least one hydrogen surrogate, the reactant composition being free from molecular H₂ and being free from base,
said liquid-assisted grinding (LAG) conditions occurring in a device chosen among: planetary ball-mill, resonant acoustic mixing (RAM), screw extrusion (single, twin or multi), drum mill, dyno-mill, or vibrating eccentric mill.

In this embodiment, the hydrogen surrogate may be either liquid or solid
In the context of the present invention, the reactant composition may comprise at least one liquid hydrogen surrogate.

In a particular embodiment, the invention concerns the use of a mechanochemical method for selectively reducing a compound containing at least one nitro, nitroso-, N-hydroxylamino group in liquid-assisted grinding (LAG) conditions into an amine, with a metal-free reactant composition comprising at least one reducing agent and at least one liquid hydrogen surrogate, the reactant composition being free from molecular H₂ and being free from base,
said liquid-assisted grinding (LAG) conditions occurring in a device chosen among: planetary ball-mill, resonant acoustic mixing (RAM), screw extrusion (single, twin or multi), drum mill, dyno-mill, or vibrating eccentric mill.

Mechanochemical methods allow to perform chemical reactions without solvents or with minimum/small amounts of solvent, and with less energy than a thermal activation.

As used herein, the terms "minimum or limited amounts of a liquid" or "minimum /small amount of solvent" refers to an eta value (η) up to 1, compared to the same reaction without mechanochemical conditions".

As used herein the expression *"metal-free reactant composition*/*condition"* refers to a composition/condition which is substantially free of metal, or preferably is totally free of metal. "Substantially free" means that the composition may comprise trace impurities (which are not catalytically effective), including metals, metal oxides and/or zeolites. For example, the composition may comprise less than 3,000 ppm (0.3 wt.%) of metal based on the total weight of the composition, less than 2,000 ppm, less than 1,000 ppm, less than 500 ppm, less than 200 ppm, less than 100 ppm, less than 50 ppm, less than 20 ppm, or even less than 1 ppm of metal.

More particularly, according to another embodiment, the reactant composition used for the synthesis of amine derivative by reduction of a compound containing at least one nitro, nitroso-, N-hydroxylamino group is metal-free of catalyst commonly used for reducing nitro-compounds such as Pd, Pt, Fe, Co, Ni, Rh, Ir, Zn and Cu.

In what precedes and follows, the "reactant composition" comprises at least one reducing agent and at least one hydrogen surrogate.

The above-mentioned reactant composition and the compound containing at least one nitro, nitroso, N-hydroxylamino group to be reduced will be designated by "the starting material mixture".

Chemical reactants are part of the reactant composition i.e reducing agent, hydrogen surrogate. Chemical reactants are also the compound to be reduced and containing at least one nitro, nitroso, N-hydroxylamino group or any additives hereafter defined.

In what precedes and follows, a "compound to be reduced" is a compound containing at least one nitro, nitroso-, N-hydroxylamino group.

### Reducing agent

As used herein, the term *"reducing agent"* refers to at least one solid reducing agent selected in the group of sodium dithionite, and other sulfur based reducing agent such as sodium hydroxymethanesulfinate, rongalite (Na⁺HOCH₂SO₂⁻ , sodium hydroxymethylsulfinate), S₈ which is cyclo-octasulphur, NaHS and Na₂S.

As used herein, the term *"reducing agent"* does not refer to the compound conventionally used in two general methods:
1. Addition of an easily oxidized metal like iron (Fe), tin (Sn) or zinc (Zn) in the presence of acid, such as HCl will convert NO₂ to NH₂.
2. Hydrogenation over a palladium, platinum, or nickel catalyst will also convert NO₂ to NH₂ with reducing agents such as H₂, and N₂H₄.

As used herein the expression *"a mechanochemical method"* refers to mechanochemistry or mechanochemical synthesis that is a technology and a research field in which solid chemical reactants, i.e in the form of granules or powders (pulverulent) or in the pasty state, are milled or ground together or ground together under mechanical activation and chemical reaction are induced by the direct absorption of mechanical energy, (McNaught, A.D.; Wilkinson, A. IUPAC Compendium of Chemical Terminology ("The Golden Book"); 2nd edition; Blackwell Scientific publications, Oxford, 1997**)** without solvents or with minimum/small amounts of solvent in a process named liquid-assisted grinding (LAG) in comparison with solution-based methods.

As used herein, the term *"liquid-assisted grinding* (LAG)" refers to minimum or limited amounts of a liquid the role of which is to increase ease of mixing and/or to participate to the reaction occurring in a mechanochemical system, for example as a solid or liquid hydrogen surrogate acting as a proton donor. Under conventionally defined LAG conditions; the liquid solvent has also been described as functioning essentially as a catalyst or lubrificant.

LAG uses a small amount of a liquid to accelerate reactions, as well as to enable and direct transformations that do not take place by neat grinding. The empirical definition of LAG is based on the way mechanochemical reactivity is affected by the ratio of the liquid solvent to the weight of chemical reactants (eta).

A value of η = 0 corresponds to neat grinding, η > 2 µL/mg corresponds to a typical solution reaction, while LAG lies in the range of ≈0-1 µL/mg. In that range, reactivity appears independent of reactant solubility, distinguishing LAG from slurry reactions (1 µL/mg < η < 2 µL/mg) in which low solubility does hinder reactivity. LAG uses a small amount of a liquid to accelerate reactions, as well as to enable and direct transformations that do not take place by neat grinding. (Friščić et al., *ACS Cent. Sci.* **2017,** 3(1), 13-19)

Solvent-free organic synthesis is eco-friendly and obviates the necessity of further steps of solvent evaporation and recycling of the solvent.

As used herein the expression *"solvent-free'* or *"substantially solvent-free*"*,* refers to the absence of any liquid solvent - or presence of limited amounts of liquid solvent.

More precisely, residual solvents, crystalline solvates, water content as residual water or bound as hydrates comprised in the composition are not meant by the feature "solvent-free", thus meaning that a *"substantially solvent-free composition*/*condition"* is a method that is substantially free from additional solvent or solvent added during or before the method.

In the context of the present invention, the reducing agent and the compound containing at least one nitro, nitroso-, N-hydroxylamino group may be solid.

According to an advantageous embodiment of the use of the invention, the reactant composition consists in a composition, that comprises at least one solid reducing agent, at least one liquid hydrogen surrogate, for selectively reducing a compound containing at least one nitro, nitroso-, N-hydroxylamino group by a mechanochemical method in liquid-assisted grinding (LAG) conditions or in solvent-free conditions, said liquid-assisted grinding (LAG) conditions occurring in a device chosen among: planetary ball-mill, resonant acoustic mixing (RAM), screw extrusion (single, twin or multi), drum mill, dyno-mill, or vibrating eccentric mill.

According to an advantageous embodiment said at least one compound containing at least one nitro, nitroso-, N-hydroxylamino group is solid.

In the context of the present invention, the reducing agent may be solid and the compound containing at least one nitro, nitroso-, N-hydroxylamino group, may be liquid.

According to an advantageous embodiment of the use of the invention, the reactant composition consists in a composition, that comprises at least one solid reducing agent, at least one liquid hydrogen surrogate, for selectively reducing a compound containing at least one nitro, nitroso-, N-hydroxylamino group by a mechanochemical method in liquid-assisted grinding (LAG) conditions or in solvent-free conditions,
said liquid-assisted grinding (LAG) conditions occurring in a device chosen among: planetary ball-mill, resonant acoustic mixing (RAM), screw extrusion (single, twin or multi), drum mill, dyno-mill, or vibrating eccentric mill.
wherein said at least one compound containing at least one nitro, nitroso-, N-hydroxylamino group is liquid.

### Device

According to an advantageous embodiment of the mechanochemical process, the mechanical method is performed with a device selected in the group of : planetary ball-mills, planetary ball-mills in multi-samples configuration (e.g., parallel mechanochemistry), drum mill, dynomill, symoloyer, or vibrating eccentric mill.

According to an advantageous embodiment of the mechanochemical process, the mechanical ball milling is performed with: Drum mill or Planetary ball-mill.

Examples of the reaction vessel and the balls as an agitation medium used in the planetary ball mill include ones formed of such materials as stainless steel, agate, alumina, tungsten carbide, chrome steel, zirconia oxide, silicon nitride and the like. Among these materials, zirconia oxide, is preferred. The size of the vessel used in the planetary ball mill is not particularly limited and may be approximately 1 cm³ to 1000 L. The size of the balls is also not particularly limited and may be approximately from 2 to 50 mm in diameter. Particularly preferred specific examples of the planetary ball mill include stainless steel and zirconia oxide as reaction vessel material.

### Resonant acoustic mixing.

Resonant acoustic mixing (RAM) involves applying a vibration to a container to induce reciprocating up and down motions on the contents of the container. The vibration may be applied with a relatively high acceleration and a relatively low amplitude. The frequency of the vibration is typically around 60 Hz. The container can be cylindrical shaped with a circumferential side wall and disc-shaped top and bottom walls. The force is applied along the longitudinal axis of the container.

In resonant acoustic mixing, acoustic energy is delivered to the components to be mixed. An oscillating mechanical driver creates motion in a mechanical system comprised engineered plates, eccentric weights and springs. This energy is then acoustically transferred to the material to be mixed. The underlying technology principle is that the system operates at resonance. In this mode, there is a nearly complete exchange of energy between the mass elements and the elements in the mechanical system.

In a resonant acoustic mixing, the only element that absorbs energy (apart from some negligible friction losses) is the reactant composition. Thus, the resonant acoustic mixing provides a highly efficient way of transferring mechanical energy directly into the starting materials (the reducing agent, the nitro-compounds and the (solid) surrogate of H₂). The resonant frequency can be from about 15 Hertz to about 2000 Hertz, or in embodiments from about 20 Hertz to about 1800 Hertz, or from about 20 Hertz to about 1700 Hertz. The g force applied by the acoustic mixer to the reactant composition can be from about 2 g force to about 100 g force.

Resonant acoustic mixers are available from Resodyn^{™} Acoustic Mixers and can be chosen from LabRAM I and LabRAM II and like.

### Screw extrusion

**Screw extrusion** may be performed at various speeds (Ss), screw temperatures (S_{T}) and residence times (T_{R}), as described herein. A single pass through the extruder may be sufficient to form the product of a mechanochemical reaction. Alternatively, when step a) is conducted in a twin-screw extruder, step a) may comprise collecting the product emerging from the twin screw extruder and subjecting it to one or more additional passes through the simple or twin-screw extruder.

Multi-screw extruders for use herein are not particularly limited. Any multi-screw extruder comprising a reaction chamber commonly known in the art may be used in the context of the present disclosure. Suitable multi-screw extruders for use herein will be easily identified by those skilled in the art, in the light of the present description.

In a particular aspect of the present application, the screw extruder for use herein is selected from the group of Single Screw Extruders (Helibar Transfer Mix, Co-Kneader), Twin Screw Extruders : 1. Intermeshing Screws: Co-rotating Parallel Screws, Counter-rotating Parallel Screws), 2: Non-intermeshing Screws (Co-rotating Parallel Screws, Co-rotating Conical Screws, Counter-rotating Screws), 3: Multiple Screw Extruders (Rotating Center Shaft: Planetary Roller Extruder), Static Center Shaft (Ring Extruder, Multiple Screw Extruder)

In one preferred aspect, the multi-screw extruder for use in the process according to the disclosure is a twin-screw extruder, in particular a co-rotating twin-screw extruder.

In an alternative execution, the multi-screw extruder for use herein is a planetary roller extruder comprising in particular a center spindle and multiple planetary gear spindles with center spindle and planetary gear spindles featuring a screw like geometry.

Selecting and fine-tuning the various operating parameters of the multi-screw extruder to the specifics of the targeted chemical reaction, is well within the capabilities of those skilled in the art. These operating parameters will be typically adapted in particular to the nature of the chemical reactants forming the starting material mixture and the targeted compound comprising a nitro-, nitroso-, hydroxylamino- group.

In one exemplary aspect, the multi-screw extruder is operated at a screw speed in a range from 10 to 2000 rpm (revolutions per minute), from 20 to 1800 rpm, from 30 to 1600 rpm, from 50 to 1500 rpm, from 50 to 1300 rpm, from 80 to 1000 rpm, from 100 to 1000 rpm, from 150 to 800 rpm, from 180 to 700 rpm, from 180 to 500 rpm, or even from 200 to 350 rpm.

In an exemplary aspect, the multi-screw extruder further comprises at least a first addition port, a second addition port, and optionally a third addition port, and the first addition stream is incorporated into the reaction chamber of the multi-screw extruder through the first addition port, the second addition stream is incorporated through the second addition port, and the optional third addition stream is incorporated through the optional third addition port.

According to a typical aspect of the process of the present disclosure, the chemical reactants, and in particular the first addition stream, the second addition stream, and the optional third addition stream are incorporated simultaneously, optionally at a different flow speed, into the reaction chamber of the multi-screw extruder. Alternatively, the chemical reactants, and in particular the first addition stream, the second addition stream, and the optional third addition stream are incorporated into the reaction chamber of the multi-screw extruder in successive steps.

In an advantageous aspect of the process, the chemical reactants, and in particular the first addition stream, the second addition stream and the optional third addition stream are such that the chemical reactants, and in particular the first, second and optionally third addition stream are liquid priorto incorporation into the reaction chamber of the multi-screw extruder.

In an advantageous aspect of the process, the chemical reactants, and in particular the first addition stream, the second addition stream and the optional third addition stream are such that the chemical reactants, and in particular the first, second and optionally third addition stream are solid prior to incorporation into the reaction chamber of the multi-screw extruder.

In an alternative aspect, the chemical reactants, and in particular the first addition stream, the second addition stream and the optional third addition stream are such that the chemical reactants, and in particular the first addition stream, the second addition stream and the optional third addition stream are at least flowable/pumpable through conventional multi-screw extruders addition pumps prior to incorporation into the reaction chamber of the multi-screw extruder.

According to a typical aspect of the process of the present disclosure, the temperature of the chemical reactants, and in particular the first addition stream, the second addition stream and the optional third addition stream is in a range from 0°C to 120°C, from 0°C to 100°C, from 0°C to 80°C, from 0°C to 70°C, from 5°C to 60°C, from 10°C to 55°C, from 15°C to 45°C, from 20°C to 35°C, or even from 20°C to 25°C, prior to incorporation into the reaction chamber of the multi-screw extruder.

In an advantageous aspect of the process, the chemical reactants, and in particular the first addition stream, the second addition stream and the optional third addition stream are incorporated into the reaction chamber of the multi-screw extruder each at a flow speed in a range from 0.1 ml/min to 500 ml/min, 0.3 ml/min to 300 ml/min, 0.5 ml/min to 200 ml/min, 0.5 ml/min to 100 ml/min, from 1 ml/ min to 80 ml/min, from 2 ml/min to 60 ml/min, or even from 2 ml/min to 50 ml/min.

In another advantageous aspect, the first addition stream and/or the optional third addition stream is incorporated into the reaction chamber of the multi-screw extruder at a flow speed in a range from 1 ml/min to 30 ml/min, from 2 ml/min to 25 ml/min, from 5 ml/min to 25 ml/min, from 5 ml/min to 20 ml/min, from 8 ml/min to 20 ml/min, or even from 10 ml/min to 20 ml/min, or for an extruder that is adapted to a laboratory: from 0.1 g/min to 10 g/min, or for larger extruders in the industry: from 1 kg/min to 50 kg/min.

The main extruder parameters to consider are:
- feed rate of chemical reactants forming the starting material mixture: from 0.1 g/min to 10 g/min for an extruder that is adapted to a laboratory (as a matter of example and in a non-limiting manner, with 9 mm, (length/diameter ratio) 25:1 TSE; from 0.4 to 1.5 g/min) and for larger extruders in the industry, from 1 kg/min to 50 kg/min)
- Number of the liquid inlet ports and their position (as a matter of example and in a non-limiting manner with ZE9 Three-Tec extruder (LD 25:1) of Three Tec company, 2 liquid ports are available: one close to the beginning of the barrel and one close to the end) used for the addition of LAG (the flow rate of LAG depends on the eta value and the feed rate of solid chemical reactants)
- Presence or not of a die and the type of die (The dies are defined and manufactured according to process requirements. Die diameters are possible from 0.1 mm. The empty volume in the die is negligible, as the screws extend into the die until just before the opening. Due to the simple design and low mass, separate die heating can be dispensed with in most cases. If heating of the die is nevertheless necessary, the same heating elements as for the barrel can be used for this purpose. The following die geometries are possible: Single and double dies, Sieve dies, Film dies (fixed or adjustable), Hose dies, Cooling dies for meat substitutes).
- Screw speed (from 20 to 1000 RPM) (as a matter of example and in a non-limiting manner Hybrid ZE9/ZE12 Three-Tec extruder of Three Tec company can rotate from 20 to 200 RPM).
- Barrel temperature (as a matter of example and in a non-limiting manner, the hybrid ZE 9/12 Three-Tec TSE extruder of Three Tec company is able to heat from 25°C to 400°C, more preferably from 25°C to 230 °C.
- Screw profile: Modular allow infinite configuration of screw profiles by replacing some screw elements by others. Screw elements can be: conveying element (reverse or not), mixing elements (reverse or not) and kneading elements.

According to a particular embodiment of the use of the invention, wherein said mechanical method is scalable and said amine is liable to be produced from about 0.1 g to about 1 kg.

As used herein the expression *"scalable"* means the ability to be changed in size or scale to industrial equipment.

According to a particular embodiment of the use of the invention, wherein said mechanical method is scalable and said amine is liable to be produced from about 0.1 g to about 500 g.

According to a particular embodiment of the use of the invention, wherein said mechanical method is scalable and said amine is liable to be produced from about 500 g to about 1 kg.

According to a particular embodiment of the use of the invention, said amine being produced in an amount greater than 10 g.

According to a particular embodiment of the use of the invention, the reactant composition comprises optionally a further additive, and the at least one compound containing at least one nitro, nitroso-, N-hydroxylamino group, and at least one reducing agent are solid.

### Other additives

Mechanochemistry reactions can require the addition of a solid additive for efficient reactivity. It has been shown that different textures of reaction mixtures can lead to different reaction kinetics, the reaction mixture can in some cases become sticky and resembles a paste, this prevents efficient mass and energy transfer. To account for this, it is common to use an additive such as silica, alumina, talc, celite or inorganic salts (e.g., NaCl) as a milling agent/auxiliary or adsorbent.

Additive can also be selected in the group of any solvent, polar or apolar (hexane, ethyl acetate, acetone, acetonitrile), bio-sourced or not, low melting polymers (e.g. low molecular weight PEGs), deep eutectic solvents (DES), including ionic liquids of any type, such as and in a non-limiting manner, DES choline chloride/glycerol (1 :2, mol/mol), also known as glyceline or choline chloride/ethylene glycol (1 : 2, mol/mol).

In the context of the present invention, the reducing agent and the compound containing at least one nitro, nitroso-, N-hydroxylamino group, may be solid, and the reactant composition may comprise an additive.

According to an advantageous embodiment of the use of the invention, the reactant composition consists in a composition, that comprises at least one solid reducing agent, at least one liquid hydrogen surrogate, and optionally a further additive for selectively reducing a compound containing at least one nitro, nitroso-, N-hydroxylamino group by a mechanochemical method in liquid-assisted grinding (LAG) conditions or in solvent-free conditions,
said liquid-assisted grinding (LAG) conditions occurring in a device chosen among: planetary ball-mill, resonant acoustic mixing (RAM), screw extrusion (single, twin or multi), drum mill, dyno-mill, or vibrating eccentric mill.
wherein said at least one compound containing at least one nitro, nitroso-, N-hydroxylamino group is solid.

In the context of the present invention, the reducing agent may be solid and the compound containing at least one nitro, nitroso-, N-hydroxylamino group may be liquid, and the reactant composition may comprise an additive.

According to an advantageous embodiment of the use of the invention, the reactant composition consists in a composition, that comprises at least one solid reducing agent, at least one liquid hydrogen surrogate, and optionally a further additive for selectively reducing a compound containing at least one nitro, nitroso-, N-hydroxylamino group by a mechanochemical method in liquid-assisted grinding (LAG) conditions or in solvent-free conditions,
said liquid-assisted grinding (LAG) conditions occurring in a device chosen among: planetary ball-mill, resonant acoustic mixing (RAM), screw extrusion (single, twin or multi), drum mill, dyno-mill, or vibrating eccentric mill.
wherein said at least one compound containing at least one nitro, nitroso-, N-hydroxylamino group is liquid.

Said additive can be either solid, liquid, or partly solid and partly liquid.

According to a particular embodiment of the use of the invention, the liquid hydrogen surrogate is a compound selected in the group of water (H₂O), an alcohol (methanol, ethanol), a polyol (ethylene glycol, diethylene glycol, triethylene glycol, propylene glycol, butylene glycol) and their mixture thereof.

As an example, and in a non-limiting manner, alcohol can also be chosen among: cyclohexanol, isopropanol, n-propanol and polyol can also be glycerol.

Polyol includes polyethylene glycol that can be liquid under mechanochemical stress. As a matter of example and in a non-limiting manner, polyol can be PEG-400, 600, 800, 1000, 2000, 3400, 5000, 20000 etc.) The end chain can be chosen among HO-PEG-OH, MeO-PEG-OMe, or HO-PEG-OMe.

According to an advantageous embodiment of the use of the invention, the liquid hydrogen surrogate is preferably water, an alcohol, and/or a polyol, their mixture thereof, preferably a mixture of EtOH:H₂O.

In the context of the present invention, the hydrogen surrogate may be H₂O.

Another particular advantageous embodiment of the use of the invention, the metal-free reactant composition comprising at least one reducing agent and H₂O for selectively reducing a compound containing at least one nitro, nitroso-, N-hydroxylamino group by a mechanochemical method in liquid-assisted grinding (LAG) conditions, the reactant composition being free from molecular H₂ and being free from base,
said liquid-assisted grinding (LAG) conditions occurring in device chosen among : planetary ball-mill, resonant acoustic mixing (RAM), screw extrusion (single, twin or multi), drum mill, dyno-mill, or vibrating eccentric mill.

According to another embodiment of the use of the invention, the volumetric ratio of said the mixture of an alcohol, and/or a polyol to H₂O is from 5:1 to 1:5, preferably from 3:1 to 1:1, more preferably is 3:1.

In the context of the present invention, the hydrogen surrogate may be a mixture of EtOH: H₂O.

Another particular advantageous embodiment, the metal-free reactant composition comprises at least one reducing agent and a mixture of EtOH: H₂O for selectively reducing a compound containing at least one nitro, nitroso-, N-hydroxylamino group by a mechanochemical method in liquid-assisted grinding (LAG) conditions, the reactant composition being free from molecular H₂ and being free from base,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

According to another advantageous embodiment of the use of the invention, the volumetric ratio of the mixture of EtOH:H₂O is from 5:1 to 1:5, preferably from 3:1 to 1:1, more preferably is 3:1.

According to an advantageous embodiment of the use of the invention, the parameter η is greater than 0 and less than 1, more preferably is comprised from about 0.1 to 0.9, more preferably from about 0.3 to 0.7, more preferably from about 0.4 to 0.6 or equal to about 0.5 or about 0.75 µL/mg.

As used herein the value for example "about 0.5" refers to said value which can vary from 5 to 10%.

According to an advantageous embodiment of the use of the invention, the reducing agent is selected in the group of sodium dithionite, and other sulfur based reducing agent such as sodium hydroxymethanesulfinate, rongalite (Na⁺HOCH₂SO₂⁻, sodium hydroxymethylsulfinate), S₈ (cyclo-octasulphur), NaHS and Na₂S.

In the context of the present invention, the hydrogen surrogate may be a mixture of EtOH: H₂O and the reducing agent may a sulfur based.

Another particular advantageous embodiment of the use of the invention, the metal-free reactant composition comprises a sulfur based reducing agent and a mixture of EtOH: H₂O for selectively reducing a compound containing at least one nitro, nitroso-, N-hydroxylamino group by a mechanochemical method in liquid-assisted grinding (LAG) conditions, the reactant composition being free from molecular H₂ and being free from base.
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In the context of the present invention, the reducing agent may be sodium dithionite.

According to an advantageous embodiment of the use of the invention, the reducing agent is sodium dithionite.

In the context of the present invention, the at least one compound containing at least one nitro, nitroso-, N-hydroxylamino group may be solid and the reducing agent may be sodium dithionite.

According to an advantageous embodiment of the use of the invention, the reactant composition consists in a composition, that comprises sodium dithionite, at least one liquid hydrogen surrogate, for selectively reducing a compound containing at least one nitro, nitroso-, N-hydroxylamino group by a mechanochemical method in liquid-assisted grinding (LAG) conditions or in solvent-free conditions,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.
wherein said at least one compound containing at least one nitro, nitroso-, N-hydroxylamino group is solid.

In the context of the present invention, the at least one compound containing at least one nitro, nitroso-, N-hydroxylamino group may be liquid and the reducing agent may be sodium dithionite.

According to an advantageous embodiment of the use of the invention, the reactant composition consists in a composition, that comprises sodium dithionite, at least one liquid hydrogen surrogate, for selectively reducing a compound containing at least one nitro, nitroso-, N-hydroxylamino group by a mechanochemical method in liquid-assisted grinding (LAG) conditions or in solvent-free conditions,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.
wherein said at least one compound containing at least one nitro, nitroso-, N-hydroxylamino group is liquid.

In the context of the present invention, , the hydrogen surrogate may be a mixture of EtOH: H₂O and the reducing agent may be sodium dithionite.

Another particular advantageous embodiment of the use the invention, the metal-free reactant composition comprises sodium dithionite and a mixture of EtOH: H₂O for selectively reducing a compound containing at least one nitro, nitroso-, N-hydroxylamino group by a mechanochemical method in liquid-assisted grinding (LAG) conditions, the reactant composition being free from molecular H₂ and being free from base,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In the context of the present invention, the hydrogen surrogate may be a mixture of EtOH: H₂O, and the reducing agent may be sodium dithionite.

Another particular advantageous embodiment of the use of the invention, the metal-free reactant composition comprising sodium dithionite and a mixture of EtOH: H₂O for selectively reducing a compound containing at least one nitro, nitroso-, N-hydroxylamino group by a mechanochemical method in liquid-assisted grinding (LAG) conditions, the reactant composition being free from molecular H₂ and being free from base, in which the volumetric ratio of the mixture EtOH:H₂O is from 3:1 to 1:1, preferably is 3:1,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In the context of the present invention, the hydrogen surrogate, the reducing agent and the compound containing at least one nitro, nitroso-, N-hydroxylamino group may be solid.

According to an advantageous embodiment of the use of the invention, the reactant composition is comprising at least one solid reducing agent and at least one solid hydrogen surrogate for selectively reducing a compound containing at least one nitro, nitroso-, N-hydroxylamino group by a mechanochemical method in liquid-assisted grinding (LAG) conditions or in solvent-free conditions,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.
wherein said at least one compound containing at least one nitro, nitroso-, N-hydroxylamino group is solid.

As an example, and in a non-limiting manner, the solid hydrogen surrogate can be Amberlite^{™} IR-120, boric acid, citric acid, NaHSO₄, NH₄Cl, B₂(OH)₄, sodium borohydride.

In the context of the present invention, the hydrogen surrogate and the reducing agent may be solid, and the compound containing at least one nitro, nitroso-, N-hydroxylamino group may be liquid.

According to an advantageous embodiment of the use of the invention, the reactant composition is comprising at least one solid reducing agent and at least one solid hydrogen surrogate for selectively reducing a compound containing at least one nitro, nitroso-, N-hydroxylamino group by a mechanochemical method in liquid-assisted grinding (LAG) conditions or in solvent-free conditions,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.
wherein said at least one compound containing at least one nitro, nitroso-, N-hydroxylamino group is liquid.

According to an advantageous embodiment, the reactant composition is free from molecular H₂ and is free from base.

According to another embodiment, the subject matter of the invention concerns a reactant composition that is solvent-free and the parameter η is equal to 0 µL/mg.

According to another embodiment, the subject matter of the invention concerns a reactant composition that contains a liquid solvent.

According to another advantageous embodiment, the optionally further additive is liquid.

According to an advantageous embodiment, the reactant composition further contains at least one solid additive selected in the group of NaCl, SiO₂, Al₂O₃, polymers (*e*.*g*., high molecular PEGs, PVA).

According to an advantageous embodiment, the reactant composition further contains at least one liquid additive selected in the group of any solvent, polar or apolar (hexane, ethyl acetate, acetone, acetonitrile), biosourced or not, low melting polymers (e.g. low molecular weight PEGs), deep eutectic solvents, including ionic liquids of any type, such as and in a non-limiting manner DES choline chloride/glycerol (1 : 2, mol/mol), also known as glyceline or choline chloride/ethylene glycol (1 : 2, mol/mol).

In the context of the present invention, the hydrogen surrogate may be different from the reducing agent

According to another embodiment of the use of the invention, the metal-free reactant composition comprises at least one reducing agent and at least one hydrogen surrogate, for selectively reducing a compound containing at least one nitro, nitroso-, N-hydroxylamino group by a mechanochemical method in liquid-assisted grinding (LAG) conditions,
said hydrogen surrogate being different from said reducing agent,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In this embodiment, an additional hydrogen surrogate is present in addition to the reducing agent.

In the context of the present invention, the solid hydrogen surrogate is different from the reducing agent

According to another embodiment of the use of the invention, the metal-free reactant composition comprises at least one reducing agent and at least one hydrogen surrogate, for selectively reducing a compound containing at least one nitro, nitroso-, N-hydroxylamino group by a mechanochemical method in liquid-assisted grinding (LAG) conditions,
said hydrogen surrogate being solid and being different from said reducing agent,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In this embodiment, an additional solid hydrogen surrogate is present in addition to the reducing agent.

In the context of the present invention, the hydrogen surrogate is different from H₂O and from the reducing agent

According to another embodiment of the use of the invention, the metal-free reactant composition comprises at least one reducing agent and at least one hydrogen surrogate, for selectively reducing a compound containing at least one nitro, nitroso-, N-hydroxylamino group by a mechanochemical method in liquid-assisted grinding (LAG) conditions,
said hydrogen surrogate being different from H₂O and being different from said reducing agent;
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In this embodiment, hydrogen surrogate can be either liquid or solid.

In the context of the present invention, the compound to be reduced may comprise a nitro group.

According to another embodiment of the use of the invention, the metal-free reactant composition comprises at least one reducing agent and at least one hydrogen surrogate, for selectively reducing a compound containing at least one nitro, group by a mechanochemical method in liquid-assisted grinding (LAG) conditions,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In the context of the present invention, the compound to be reduced may comprise a nitroso group.

According to another embodiment of the use the of the invention, the metal-free reactant composition comprises at least one reducing agent and at least one hydrogen surrogate, for selectively reducing a compound containing at least one nitroso, group by a mechanochemical method in liquid-assisted grinding (LAG) conditions,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In the context of the present invention, the compound to be reduced may comprise a N-hydroxylamino group.

According to another embodiment of the use of the invention, the metal-free reactant composition comprises at least one reducing agent and at least one hydrogen surrogate, for selectively reducing a compound containing at least one N-hydroxylamino, group by a mechanochemical method in liquid-assisted grinding (LAG) conditions,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

### The nitro, nitroso-, N-hydroxylamino compounds (the "Ar-Y" compound)

According to the present invention of the use of the invention, a compound containing at least one nitro, nitroso-, N-hydroxylamino group is a substituted or unsubstituted aromatic or heteroaromatic compound.

In the context of the present invention, the compound to be reduced may be a compound of general formula Ar-Y.

More preferably, said compound is advantageously chosen from a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions.

In the context of the present invention, Ar may be a simple aromatic ring.

According to another embodiment of the use of the invention, Ar is a simple aromatic ring, optionally a heterocyclic containing an atom of oxygen, nitrogen, or sulfur.

In the context of the present invention, Ar may be a carbon aromatic ring.

According to another embodiment of the use of the invention, Ar is a monocyclic as in benzene, bicyclic as in naphthalene, or polycyclic as in anthracene.

In the context of the present invention, Ar may be a 5 membered aromatic ring.

According to another embodiment, Ar is a five-membered ring like pyrrole or six-membered ring like pyridine.

According to another embodiment of the use of the invention, Ar is chosen among the following compounds: Furan, Benzofuran, Isobenzofuran, Pyrrole, Indole, Isoindole, Thiophene, Benzothiophene, Imidazole, Benzimidazole, Purine, Pyrazole, Indazole, Oxazole, Benzoxazole, Isoxazole, Benzisoxazole, Thiazole, Benzothiazole, Benzene, Naphthalene, Anthracene, Pyridine, Quinoline, Isoquinoline, Pyrazine, Quinoxaline, Acridine, Pyrimidine, Quinazoline, Pyridazine, Cinnoline, Phthalazine, 1,2,3-Triazine, 1,2,4-Triazine, with or without one or more substitutions.

In the context of the present invention, Ar may be substituted by a group which also may be reduced.

According to another embodiment of the use of the invention, Ar is substituted by at least one group which can also be reduced.

In the context of the present invention, Ar may be substituted by a chain which is also substituted by a group that may be reduced.

According to another embodiment of the use of the invention, Ar is substituted by a linear, branched or cyclo alkyl group, substituted with at least one group which can also be reduced.

In the context of the present invention, Ar may be substituted by a chain which is also substituted by a group that cannot be reduced.

According to another embodiment of the use of the invention, Ar is substituted by a group chosen among the following groups: aldehyde, nitrile, ester and ketone; said groups cannot be reduced.

In the context of the present invention, the hydrogen surrogate may be liquid and the compound to be reduced may be a compound of general formula Ar-Y.

A particular advantageous embodiment of the use of the invention, the metal-free reactant composition comprising at least one reducing agent and at least one liquid hydrogen surrogate for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions, by a mechanochemical method in liquid-assisted grinding (LAG) conditions, the reactant composition being free from molecular H₂ and being free from base.
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In the context of the present invention, hydrogen surrogate may be H₂O and the compound to be reduced may be a compound of general formula Ar-Y.

Another particular advantageous embodiment of the use of the invention, the metal-free reactant composition comprises at least one reducing agent and H₂O for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions, by a mechanochemical method in liquid-assisted grinding (LAG) conditions, the reactant composition being free from molecular H₂ and being free from base,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In the context of the present invention, hydrogen surrogate may be a mixture of EtOH: H₂O and the compound to be reduced may be a compound of general formula Ar-Y.

Another particular advantageous embodiment of the use of the invention, the metal-free reactant composition comprises at least one reducing agent and a mixture of EtOH: H₂O for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions, by a mechanochemical method in liquid-assisted grinding (LAG) conditions, the reactant composition being free from molecular H₂ and being free from base,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In the context of the present invention, hydrogen surrogate may be a mixture of EtOH: H₂O, the reducing agent may be sulfur based and the compound to be reduced may be a compound of general formula Ar-Y.

Another particular advantageous embodiment of the use of the invention, the metal-free reactant composition comprises a sulfur based reducing agent and a mixture of EtOH: H₂O for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions, by a mechanochemical method in liquid-assisted grinding (LAG) conditions, the reactant composition being free from molecular H₂ and being free from base,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In the context of the present invention, hydrogen surrogate may be a mixture of EtOH: H₂O, the reducing agent may be sodium dithionite and the compound to be reduced may be a compound of general formula Ar-Y.

Another particular advantageous embodiment of the use of the invention, the metal-free reactant composition comprises sodium dithionite and a mixture of EtOH: H₂O for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions, by a mechanochemical method in liquid-assisted grinding (LAG) conditions, the reactant composition being free from molecular H₂ and being free from base,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In the context of the present invention, hydrogen surrogate may be a mixture of EtOH: H₂O, the reducing agent may be sodium dithionite and the compound to be reduced may be a compound of general formula Ar-Y.

Another particular advantageous embodiment of the use the invention, the metal-free reactant composition comprising sodium dithionite and a mixture of EtOH: H₂O for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions, by a mechanochemical method in liquid-assisted grinding (LAG) conditions, the reactant composition being free from molecular H₂ and being free from base, in which the volumetric ratio of the mixture EtOH:H₂O is from 3:1 to 1:1, preferably is 3:1,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

Another particular advantageous embodiment of the use of the invention, the metal-free reactant composition comprises sodium dithionite and a mixture of EtOH: H₂O for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions, by a mechanochemical method in liquid-assisted grinding (LAG) conditions, the reactant composition being free from molecular H₂ and being free from base, in which the volumetric ratio of the mixture EtOH:H₂O is from 3:1 to 1:1, preferably is 3:1 and in which the parameter η is comprised from about 0 to 1, more preferably from about 0.3 to 0.7 or equal to about 0.5 or about 0.75 µL/mg ,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

In the context of the process according to the invention, the hydrogen surrogate may be liquid or solid and the process may comprise one step.

The invention also concerns a mechanochemical process which comprises the step of :
mixing, milling and/or grinding a starting material mixture of at least one reducing agent, at least one solid or liquid hydrogen surrogate and at least one compound containing at least one nitro, nitroso, N-hydroxylamino group, which includes no metal catalyst, no molecular H₂ and no base, in a reacting environment subjected to a mechanical force to generate a mechanochemical reaction between the at least one reducing agent, at least one solid or liquid hydrogen surrogate and at least one compound containing at least one nitro, nitroso, N-hydroxylamino group, to obtain an amine derivative
said mechanical force occurring in a device chosen among: planetary ball-mill, resonant acoustic mixing (RAM), screw extrusion (single, twin or multi), drum mill, dyno-mill, or vibrating eccentric mill.

In the context of the process according to the invention, the process may comprise two steps.

According to another embodiment of the process of the invention, the mechanochemical process comprises the steps of :
a) preparing or providing a starting material mixture of at least one reducing agent, at least one solid or liquid hydrogen surrogate and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between the at least one reducing agent, at least one solid or liquid hydrogen surrogate and at least one compound containing at least one nitro, nitroso, N-hydroxylamino group, to obtain an amine derivative,
said mechanical force occurring in a device chosen among: planetary ball-mill, resonant acoustic mixing (RAM), screw extrusion (single, twin or multi), drum mill, dyno-mill, or vibrating eccentric mill.

In the context of the present invention, the reducing agent may be solid.

According to an advantageous embodiment of the process of the invention, the at least one compound containing at least one nitro, nitroso-, N-hydroxylamino group and at least one reducing agent are solid.

In the context of the process according to the invention, the reducing agent may be solid and the compound containing at least one nitro, nitroso-, N-hydroxylamino group may be solid.

According to an advantageous embodiment of the process of the invention, the at least one compound containing at least one nitro, nitroso-, N-hydroxylamino group is solid and at least one reducing agent is solid.

Another object of the present invention relates to a mechanochemical process for selectively reducing a compound containing at least one nitro, nitroso, N-hydroxylamino group to an amine derivative by reacting the said compound to be reduced with a reactant composition comprising at least one reducing agent and at least one hydrogen surrogate in liquid-assisted grinding (LAG) conditions or in solvent-free conditions and in the absence of a catalyst metal, in the absence of molecular H₂ and in the absence of a base.

In the context of the process according to the invention, the hydrogen surrogate may be liquid.

Another embodiment of the present invention relates to a mechanochemical process for selectively reducing a compound to be reduced to an amine derivative by reacting the said compound to be reduced with at least one reducing agent and at least one liquid hydrogen surrogate in liquid-assisted grinding (LAG) conditions and in the absence of a catalyst metal, in the absence of molecular H₂ and in the absence of a base,
said liquid-assisted grinding (LAG) conditions occurring in a device chosen among : planetary ball-mill, resonant acoustic mixing (RAM), screw extrusion (single, twin or multi), drum mill, dyno-mill, or vibrating eccentric mill.

As stated hereinabove, the reaction proceeds with a chemoselectivity with complete or substantially complete conversion of nitro, nitroso, N-hydroxylamino group to an amine derivative, e.g. > 60 %, more particularly > 70%, more particularly > 80%, more particularly > 90%, and still more particularly 95-100 %, of the nitro, nitroso, N-hydroxylamino functionality is converted.

The process of the present application is chemoselective, as it reduces nitro groups without reducing other functional groups present in the compound containing at least one nitro, nitroso, N-hydroxylamino group, in the presence of other groups which can also be reduced.

According to another embodiment, the compound containing at least one nitro, nitroso, N-hydroxylamino group can also comprise a group chosen among the following groups: aldehyde, nitrile, ester and ketone.

The process of the present application is chemoselective towards aldehyde, nitrile, ester and ketone.

In the context of the process according to the invention, the hydrogen surrogate may be liquid and the process may comprise one step.

According to another embodiment of the process of the invention, the mechanochemical process comprises the step of :
mixing, milling and/or grinding a starting material mixture of at least one reducing agent, at least one liquid hydrogen surrogate and at least one compound containing at least one nitro, nitroso, N-hydroxylamino group, which includes no metal catalyst, in a reacting environment subjected to a mechanical force to generate a mechanochemical reaction between the at least one reducing agent, at least one solid or liquid hydrogen surrogate and at least one compound containing at least one nitro, nitroso, N-hydroxylamino group, to obtain an amine derivative.

In the context of the process according to the invention, the hydrogen surrogate may be liquid and the process may comprise two steps.

According to another embodiment of the process of the invention, the mechanochemical process comprises the steps of :
a) preparing or providing a starting material mixture of at least one reducing agent, at least one liquid hydrogen surrogate and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between the at least one reducing agent, at least one liquid hydrogen surrogate and at least one compound containing at least one nitro, nitroso, N-hydroxylamino group, to obtain an amine derivative.

As used herein, the expression " *mechanical force* " refers to any kind of energy that would be sufficient as activation energy to start a chemical reaction process. In a preferred embodiment the chemically stimulating energy is kinetic energy, preferably kinetic energy produced by a ball mill. Kinetic energy is generated by at least one ball in the ball mill. In the process according to the invention ball mill might then be considered as being the active reaction container and a ball of the ball mill might then be considered as being the part of the active reaction container. In another preferred embodiment the chemically stimulating energy is produced by RAM, TSE (Twin screw extrusion).

In the context of the process according to the invention, the hydrogen surrogate may be liquid and the reducing agent may be solid.

According to an advantageous embodiment of the process of the invention, the at least one hydrogen surrogate is liquid and at least one reducing agent is solid.

In the context of the process according to the invention, the hydrogen surrogate may be liquid and the compound containing at least one nitro, nitroso-, N-hydroxylamino group may be solid.

According to an advantageous embodiment of the process of the invention, the at least one compound containing at least one nitro, nitroso-, N-hydroxylamino group is solid and the at least one hydrogen surrogate is liquid and at least one reducing agent is solid.

In the context of the process according to the invention, the hydrogen surrogate may be liquid and the compound containing at least one nitro, nitroso-, N-hydroxylamino group may be liquid.

According to an advantageous embodiment of the process of the invention, the at least one compound containing at least one nitro, nitroso-, N-hydroxylamino group is liquid and the at least one hydrogen surrogate is liquid and at least one reducing agent is liquid.

In the context of the process according to the invention, the hydrogen surrogate may be liquid.

According to another embodiment of the process of the invention, the mechanochemical process comprises the steps of:
a) preparing or providing a starting material mixture of at least one reducing agent, at least one liquid hydrogen surrogate and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between the at least one reducing agent, at least one liquid hydrogen surrogate and at least one compound containing at least one nitro, nitroso, N-hydroxylamino group, to obtain an amine derivative, and in which the LAG conditions are defined by the parameter η that is greater than 0 and less than 1, more preferably is comprised from about 0.1 to 0.9, more preferably from about 0.3 to 0.7, more preferably from about 0.4 to 0.6 or equal to about 0.5 or about 0.75 µL/mg,
said mechanical force occurring in a device chosen among: planetary ball-mill, resonant acoustic mixing (RAM), screw extrusion (single, twin or multi), drum mill, dyno-mill, or vibrating eccentric mill.

According to an advantageous embodiment of the process of the invention, the said starting material mixture comprises optionally a further additive.

In the context of the process according to the invention, the hydrogen surrogate may be liquid.

According to a particular embodiment of the process of the invention, the hydrogen surrogate is a liquid compound selected in the group of water (H₂O), an alcohol (methanol, ethanol), a polyol (ethylene glycol, diethylene glycol, triethylene glycol, propylene glycol, butylene glycol) and their mixture thereof.

As an example, and in a non-limiting manner, alcohol can also be chosen among: cyclohexanol, i-propanol, n-propanol and polyol can also be glycerol.

Polyol includes polyethylene glycol that can be liquid under mechanochemical stress. As a matter of example and in a non-limiting manner, polyol can be PEG-(400, 600, 800, 1000, 2000, 3400, 5000, 20000 etc.) The end chain can be selected among HO-PEG-OH, MeO-PEG-OMe, or HO-PEG-OMe..

According to an advantageous embodiment of the process of the invention, wherein said process is scalable and said amine is liable to be produced from about 0.1 g to about 1 kg.

According to an advantageous embodiment of the process of the invention, wherein said process is scalable and said amine is liable to be produced from about 0.1 g to about 500 g.

According to an advantageous embodiment of the process of the invention, wherein said process is scalable and said amine is liable to be produced from 500 g to 1 kg.

According to an advantageous embodiment of the process of the invention, wherein said amine is produced in an amount greater than 10 g.

According to an advantageous embodiment of the process of the invention, the hydrogen surrogate is preferably water, an alcohol, and/or a polyol, their mixture thereof, preferably a mixture of EtOH:H₂O.

According to another embodiment of the process of the invention, the weight ratio of said the mixture of an alcohol, and/or a polyol to H₂O is from 5:1 to 1:5, preferably from 3:1 to 1:1, more preferably is 3:1.

In the context of the process according to the invention, the hydrogen surrogate may be H₂O.

Another particular advantageous embodiment is a mechanochemical process for selectively reducing a compound containing at least one nitro, nitroso, N-hydroxylamino group to an amine derivative by reacting the said compound containing at least one nitro, nitroso, N-hydroxylamino group with at least one reducing agent and H₂O in liquid-assisted grinding (LAG) conditions and in the absence of a catalyst metal, in the absence of molecular H₂ and in the absence of a base, comprising
a) preparing or providing a starting material mixture of at least one reducing agent, H₂O and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between the at least one reducing agent, H₂O and at least one compound containing at least one nitro, nitroso, N-hydroxylamino group, to obtain an amine derivative,
said mechanical force occurring in a device chosen among : planetary ball-mill, resonant acoustic mixing (RAM), screw extrusion (single, twin or multi), drum mill, dyno-mill, or vibrating eccentric mill.

In the context of the process according to the invention, the hydrogen surrogate may be a mixture of H₂O and EtOH.

According to an advantageous embodiment of the process of the invention, the hydrogen surrogate is a mixture of EtOH:H₂O.

According to another advantageous embodiment of the process of the invention, the weight ratio of the mixture of EtOH:H₂O is from 5:1 to 1:5, preferably from 3:1 to 1:1, more preferably is 3:1.

According to another advantageous embodiment of the process of the invention, in which the liquid hydrogen surrogate is preferably water, an alcohol, and/or a polyol, their mixture thereof, preferably a mixture of EtOH:H₂O,
or,
in which the liquid hydrogen surrogate is a mixture of EtOH:H₂O and the weight ratio of said mixture of EtOH:H₂O is from 5:1 to 1:5, preferably from 3:1 to 1:1, more preferably is 3:1.

In the context of the process according to the invention, the reducing agent may be sulfur based.

According to an advantageous embodiment of the process of the invention, the reducing agent is selected in the group of sodium dithionite, and other sulfur based reducing agent such as sodium hydroxymethanesulfinate, rongalite (Na⁺HOCH₂SO₂⁻, sodium hydroxymethylsulfinate), S8 (cyclo-octasulphur), NaHS and Na₂S.

In the context of the process according to the invention, the hydrogen surrogate may be a mixture of EtOH : H₂O, and the reducing agent may be sulfur based.

**Another particular advantageous embodiment is** a mechanochemical process for selectively reducing a compound containing at least one nitro, nitroso, N-hydroxylamino group to an amine derivative by reacting the said compound containing at least one nitro, nitroso, N-hydroxylamino group with a sulfur based reducing agent and a mixture of EtOH: H₂O in liquid-assisted grinding (LAG) conditions and in the absence of a catalyst metal, in the absence of molecular H₂, and in the absence of a base, comprising
a) preparing or providing a starting material mixture of a sulfur based reducing agent, a mixture of EtOH: H₂O and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between the sulfur based reducing agent, the mixture of EtOH: H₂O and at least one compound containing at least one nitro, nitroso, N-hydroxylamino group, to obtain an amine derivative,
said mechanical force occurring in a device chosen among : planetary ball-mill, resonant acoustic mixing (RAM), screw extrusion (single, twin or multi), drum mill, dyno-mill, or vibrating eccentric mill.

In the context of the process according to the invention, the reducing agent may be sodium dithionite.

According to an advantageous embodiment of the process of the invention, the reducing agent is sodium dithionite.

In the context of the process according to the invention, the hydrogen surrogate may be a mixture of EtOH : H₂O, and the reducing agent may be sodium dithionite.

Another particular advantageous embodiment is a mechanochemical process for selectively reducing a compound containing at least one nitro, nitroso, N-hydroxylamino group to an amine derivative by reacting the said compound containing at least one nitro, nitroso, N-hydroxylamino group with sodium dithionite and a mixture of EtOH: H₂O in liquid-assisted grinding (LAG) conditions and in the absence of a catalyst metal, in the absence of molecular H₂ and in the absence of a base, comprising
a) preparing or providing a starting material mixture of sodium dithionite, a mixture of EtOH: H₂O and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between sodium dithionite, the mixture of EtOH: H₂O and at least one compound containing at least one nitro, nitroso, N-hydroxylamino group, to obtain an amine derivative,
said mechanical force occurring in a device chosen among : planetary ball-mill, resonant acoustic mixing (RAM), screw extrusion (single, twin or multi), drum mill, dyno-mill, or vibrating eccentric mill.

In the context of the process according to the invention, the hydrogen surrogate may be a mixture of EtOH : H₂O and the reducing agent may be sodium dithionite.

Another particular advantageous embodiment is a mechanochemical process for selectively reducing a compound containing at least one nitro, nitroso, N-hydroxylamino group to an amine derivative by reacting the said compound containing at least one nitro, nitroso, N-hydroxylamino group with sodium dithionite and a mixture of EtOH: H₂O in liquid-assisted grinding (LAG) conditions and in the absence of a catalyst metal, in the absence of molecular H₂ and in the absence of a base, comprising
a) preparing or providing a starting material mixture of sodium dithionite, a mixture of EtOH: H₂O and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between sodium dithionite, the mixture of EtOH: H₂O and at least one compound containing at least one nitro, nitroso, N-hydroxylamino group, to obtain an amine derivative,

said mechanical force occurring in a device chosen among : planetary ball-mill, resonant acoustic mixing (RAM), screw extrusion (single, twin or multi), drum mill, dyno-mill, or vibrating eccentric mill.
in which the volumetric ratio of the mixture EtOH:H₂O is from 3:1 to 1:1, preferably is 3:1.

In the context of the process according to the invention, the hydrogen surrogate may be a mixture of H₂O and the reducing agent may be sodium dithionite.

**Another particular advantageous embodiment is** a mechanochemical process for selectively reducing a compound containing at least one nitro, nitroso, N-hydroxylamino group to an amine derivative by reacting the said compound containing at least one nitro, nitroso, N-hydroxylamino group with sodium dithionite and a mixture of EtOH: H₂O in liquid-assisted grinding (LAG) conditions and in the absence of a catalyst metal, in the absence of molecular H₂ and in the absence of a base, comprising
a) preparing or providing a starting material mixture of sodium dithionite, a mixture of EtOH: H₂O and at least one compound containing at least one nitro, nitroso, N-hydroxylamino group, and/or
b) mixing, milling and/or grinding said mixture, which includes no metal catalyst, in a reacting environment subjected to a mechanical force to generate a mechanochemical reaction between sodium dithionite, the mixture of EtOH: H₂O and at least one compound containing at least one nitro, nitroso, N-hydroxylamino group, to obtain an amine derivative,

said mechanical force occurring in a device chosen among : planetary ball-mill, resonant acoustic mixing (RAM), screw extrusion (single, twin or multi), drum mill, dyno-mill, or vibrating eccentric mill.
in which the volumetric ratio of the mixture EtOH:H₂O is from 3:1 to 1:1, preferably is 3:1, and
in which the parameter η is comprised from about 0.3 to 1,2, more preferably from about 0.5 to 1 or equal to about 0.5 or about 0.75 µL/mg.

### The nitro, nitroso-, N-hydroxylamino compounds (the "Ar-Y" compound)

According to a process of the invention, a compound containing at least one nitro, nitroso-, N-hydroxylamino group is a substituted or unsubstituted aromatic or heteroaromatic compound.

In the context of the process according to the invention, the compound to be reduced may be of general formula Ar-Y.

More preferably, according to a process of the invention, said compound is advantageously chosen from a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions.

In the context of the process according to the invention, Ar may be a simple aromatic ring.

According to another embodiment of the process of the invention, Ar is a simple aromatic ring, optionally a heterocyclic containing, an atom of oxygen, nitrogen, or sulfur.

In the context of the process according to the invention, Ar may be a carbon aromatic ring.

According to another embodiment of the process of the invention, Ar is a monocyclic as in benzene, bicyclic as in naphthalene, or polycyclic as in anthracene.

In the context of the process according to the invention, Ar may be a 5 membered aromatic ring.

According to another embodiment of the process of the invention, Ar is a five-membered ring like pyrrole or six-membered ring like pyridine.

According to another embodiment of the process of the invention, Ar is chosen among the following compounds: Furan, Benzofuran, Isobenzofuran, Pyrrole, Indole, Isoindole, Thiophene, Benzothiophene, Imidazole, Benzimidazole, Purine, Pyrazole, Indazole, Oxazole, Benzoxazole, Isoxazole, Benzisoxazole, Thiazole, Benzothiazole, Benzene, Naphthalene, Anthracene, Pyridine, Quinoline, Isoquinoline, Pyrazine, Quinoxaline, Acridine, Pyrimidine, Quinazoline, Pyridazine, Cinnoline, Phthalazine, 1,2,3-Triazine, 1,2,4-Triazine, with or without one or more substitutions.

According to another embodiment of the process of the invention, Ar is substituted by at least one group which can also be reduced.

In the context of the process according to the invention, Ar may be substituted by a chain, also substituted with a group that may be reduced.

According to another embodiment of the process of the invention, Ar is substituted by a linear, branched or cyclo alkyl group, substituted with at least one group which can also be reduced.

In the context of the process according to the invention, Ar may be substituted by a group that cannot be reduced.

According to another embodiment of the process of the invention, Ar is substituted by a group chosen among the following groups: aldehyde, nitrile, ester and ketone; said groups cannot be reduced.

According to another embodiment of the process of the invention, the molar ratio of the Ar-Y compound to the reducing agent is from 1: 2 to 2 : 1, preferably from 1: 2 to 1: 10, more preferably from 1: 2 to 1: 6, even more preferably from 1: 3 to 1: 4.

In the context of the process according to the invention, the compound to be reduced may be of general formula Ar-Y and the process can comprise one step.

A particular advantageous embodiment is a mechanochemical process for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions to an amine derivative by reacting at least one reducing agent and at least one hydrogen surrogate in liquid-assisted grinding (LAG) conditions or in solvent-free conditions and in the absence of a catalyst metal, in the absence of molecular H₂ and in the absence of a base, comprising
a) preparing or providing a starting material mixture of at least one reducing agent, at least one solid or liquid hydrogen surrogate and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between the at least one reducing agent, at least one solid or liquid hydrogen surrogate and at least one compound containing at least one nitro, nitroso, N-hydroxylamino group, to obtain an amine derivative,
said mechanical force occurring in a device chosen among : planetary ball-mill, resonant acoustic mixing (RAM), screw extrusion (single, twin or multi), drum mill, dyno-mill, or vibrating eccentric mill.

In the context of the process according to the invention, the compound to be reduced may be of general formula Ar-Y and the hydrogen surrogate may be liquid.

Another particular advantageous embodiment is a mechanochemical process for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions to an amine derivative by reacting at least one reducing agent and at least one liquid hydrogen surrogate in liquid-assisted grinding (LAG) conditions and in the absence of a catalyst metal, in the absence of molecular H₂ and in the absence of a base, comprising
a) preparing or providing a starting material mixture of at least one reducing agent, at least one solid or liquid hydrogen surrogate and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between the at least one reducing agent, at least one liquid hydrogen surrogate and at least one compound containing at least one nitro, nitroso, N-hydroxylamino group, to obtain an amine derivative,
said mechanical force occurring in a device chosen among : planetary ball-mill, resonant acoustic mixing (RAM), screw extrusion (single, twin or multi), drum mill, dyno-mill, or vibrating eccentric mill.

In the context of the process according to the invention, the compound to be reduced may be of general formula Ar-Y and the hydrogen surrogate may be H₂O.

Another particular advantageous embodiment is a mechanochemical process for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions to an amine derivative by reacting at least one reducing agent and H₂O in liquid-assisted grinding (LAG) conditions and in the absence of a catalyst metal, in the absence of molecular H₂ and in the absence of a base, comprising
a) preparing or providing a starting material mixture of at least one reducing agent, at least one solid or liquid hydrogen surrogate and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between the at least one reducing agent, H₂O and at least one compound containing at least one nitro, nitroso, N-hydroxylamino group, to obtain an amine derivative,
said mechanical force occurring in a device chosen among : planetary ball-mill, resonant acoustic mixing (RAM), screw extrusion (single, twin or multi), drum mill, dyno-mill, or vibrating eccentric mill.

In the context of the process according to the invention, the compound to be reduced may be of general formula Ar-Y and the hydrogen surrogate may be a mixture of EtOH: H₂O.

Another particular advantageous embodiment is a mechanochemical process for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions to an amine derivative by reacting at least one reducing agent and a mixture of EtOH: H₂O in liquid-assisted grinding (LAG) conditions and in the absence of a catalyst metal, in the absence of molecular H₂, and in the absence of a base comprising
a) preparing or providing a starting material mixture of at least one reducing agent, at least one solid or liquid hydrogen surrogate and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between the at least one reducing agent, a mixture of EtOH: H₂O and at least one compound containing at least one nitro, nitroso, N-hydroxylamino group, to obtain an amine derivative,
said mechanical force occurring in a device chosen among : planetary ball-mill, resonant acoustic mixing (RAM), screw extrusion (single, twin or multi), drum mill, dyno-mill, or vibrating eccentric mill.

In the context of the process according to the invention, the compound to be reduced may be of general formula Ar-Y, the hydrogen surrogate may be a mixture of EtOH: H₂O and the reducing agent may be sulfur based.

Another particular advantageous embodiment is a mechanochemical process for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions to an amine derivative by reacting a sulfur based reducing agent and a mixture of EtOH: H₂O in liquid-assisted grinding (LAG) conditions and in the absence of a catalyst metal, in the absence of molecular H₂ and in the absence of a base, comprising
a) preparing or providing a starting material mixture of a sulfur based reducing agent, a mixture of EtOH: H₂O and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between the sulfur based reducing agent, the mixture of EtOH: H₂O and at least one compound containing at least one nitro, nitroso, N-hydroxylamino group, to obtain an amine derivative,
said mechanical force occurring in a device chosen among: planetary ball-mill, resonant acoustic mixing (RAM), screw extrusion (single, twin or multi), drum mill, dyno-mill, or vibrating eccentric mill.

In the context of the process according to the invention, the compound to be reduced may be of general formula Ar-Y, the hydrogen surrogate may be a mixture of EtOH: H₂O and the reducing agent may be sodium dithionite.

Another particular advantageous embodiment is a mechanochemical process for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions to an amine derivative by reacting sodium dithionite and a mixture of EtOH: H₂O in liquid-assisted grinding (LAG) conditions and in the absence of a catalyst metal, in the absence of molecular H₂ and in the absence of a base, comprising
a) preparing or providing a starting material mixture of sodium dithionite, a mixture of EtOH: H₂O and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between sodium dithionite, the mixture of EtOH: H₂O and at least one compound containing at least one nitro, nitroso, N-hydroxylamino group, to obtain an amine derivative,
said mechanical force occurring in a device chosen among : planetary ball-mill, resonant acoustic mixing (RAM), screw extrusion (single, twin or multi), drum mill, dyno-mill, or vibrating eccentric mill.

In the context of the process according to the invention, the compound to be reduced may be of general formula Ar-Y, the hydrogen surrogate may be a mixture of EtOH: H₂O, and the reducing agent may be sodium dithionite.

Another particular advantageous embodiment is a mechanochemical process for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions to an amine derivative by reacting sodium dithionite and a mixture of EtOH: H₂O in liquid-assisted grinding (LAG) conditions and in the absence of a catalyst metal, in the absence of molecular H₂ and in the absence of a base, comprising
a) preparing or providing a starting material mixture of sodium dithionite, a mixture of EtOH: H₂O and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between sodium dithionite, the mixture of EtOH: H₂O and at least one compound containing at least one nitro, nitroso, N-hydroxylamino group, to obtain an amine derivative,

said mechanical force occurring in a device chosen among : planetary ball-mill, resonant acoustic mixing (RAM), screw extrusion (single, twin or multi), drum mill, dyno-mill, or vibrating eccentric mill.
in which the volumetric ratio of the mixture EtOH:H₂O is from 3:1 to 1:1, preferably is 3:1.

In the context of the process according to the invention, the compound to be reduced may be of general formula Ar-Y, the hydrogen surrogate may be a mixture of EtOH: H₂O, and the reducing agent may be sodium dithionite.

Another particular advantageous embodiment is a mechanochemical process for selectively reducing a substituted or unsubstituted nitroaromatic, nitrosoaromatic and/or aromatic hydroxylamine of general formula Ar-Y, wherein Y is NO₂, NO or NHOH and Ar is an aromatic or a heteroaromatic ring with or without one or more substitutions to an amine derivative by reacting sodium dithionite and a mixture of EtOH: H₂O in liquid-assisted grinding (LAG) conditions and in the absence of a catalyst metal, in the absence of molecular H₂ and in the absence of a base, comprising
a) preparing or providing a starting material mixture of sodium dithionite, a mixture of EtOH: H₂O and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between sodium dithionite, the mixture of EtOH: H₂O and at least one compound containing at least one nitro, nitroso, N-hydroxylamino group, to obtain an amine derivative,

said mechanical force occurring in a device chosen among : planetary ball-mill, resonant acoustic mixing (RAM), screw extrusion (single, twin or multi), drum mill, dyno-mill, or vibrating eccentric mill.
in which the volumetric ratio of the mixture EtOH:H₂O is from 3:1 to 1:1, preferably is 3:1, and in which the parameter η is comprised from about 0.3 to 1,2, more preferably from about 0.5 to 1 or equal to about 0.5 or about 0.75 µL/mg.

According to another embodiment, the at least one reducing agent and of at least one compound containing at least one nitro, nitroso, N-hydroxylamino group can be pre-milled or pre-grinded separately and mixed together before adding at least one liquid hydrogen surrogate.

The mechanochemical reactions of the process of the present invention can be made by various methods. As will be easily apparent to those skilled in the art, the devices for use herein may comprise various addition ports for the incorporation of various reactant/reagent addition streams into the reaction chamber. The various reactant/reagent addition streams may be incorporated into the reaction chamber through distinct or common addition ports. Similarly, the various reactant/reagent addition streams may be incorporated into the reaction chamber simultaneously or at distinct addition times. Similarly, the various reactant/reagent addition streams may be incorporated into the reaction chamber at equal or dissimilar flow speeds.

In the process according to the invention, the magnitude of the mechanical energy necessary to bring about the mechanochemical synthesis of the amine derivative by reducing a compound containing at least one nitro, nitroso-, N-hydroxylamino group will depend on various parameters, in particular on the modulation of technical and process parameters specific to the mechanochemical process, which allow to deliver the suitable mechanical energy to the reacting system. The mechanochemical parameters (technical and process variables) depends on the type of equipment used.

The energy necessary for the mechanochemical synthesis can be passed on to the reactants via attritional forces, shear forces, compression forces' (as in the case of a presence of the extruder), impact forces (as in the case of ball-mills), friction forces (as in the case of RAM and ball-mill) or via a combination of these forces. The process can be carried out in any appropriate known device capable of generating the attritional and/or shear energy necessary.

In the process according to the invention, the mechanochemical synthesis is carried out at standard atmospheric pressure.

The optimum temperature will depend on various parameters, including the selection of the materials participating in the mixture (M) and their physical state.

The choice is preferably made of a temperature of less than or equal to 100°C and of greater than or equal to 0°C and particularly preferably a temperature of less than or equal to 80°C and of greater than or equal to 10°C. Very particularly preferably, the starting temperature is the room temperature and the latter naturally rises during the reaction to reach values of the order of 50°C maximum.

In the process according to the invention, the duration of grinding/miling advantageously varies between a few minutes and a few hours depending on the nature of the compound containing at least one nitro, nitroso-, N-hydroxylamino group, of the reducing agent and of the presence or the absence of solvent (liquid or solid) or any further additive.

Preferably, the duration is at least 5 minutes, particularly preferably at least 10 minutes and very particularly preferably at least 15 minutes. Preferably, the duration is at most 10 hours, particularly preferably at most 5 hours and very particularly preferably at most 2 hours. Durations of between 20 minutes and 1 hour are highly suitable.

If both reactive compounds are solids, they can be ground separately before the mechanochemical process begins.

In the case of using a RAM, optionally, if possible, a slight increase of the temperature to produce a homogeneous mixture would be convenient. The starting materials are combined at slow speed and then ground together at higher speed. The grinding can be done under solvent-free or LAG conditions.

The energy of the grinding process can be varied within wide ranges. Low energy ball-milling, attrition milling, vibratory milling and similar low energy grinding processes known in the art of grinding tend to be preferred over high energy milling processes, since the use of a grinding medium at high energy can produce wear and, thereby, contaminate the reactant and the product obtained. However, when high energy milling is needed, high energy ball milling, high energy planetary milling and similar can be used. Grinding media that do not react with the reactant compound and product are preferred and can be agate or similar materials.

By way of example and in a non-limiting manner, the material forming the grinding media can be chosen among: agate, stainless steel, zirconium oxide, PTFE (polytetrafluoroethylene), POM (polyoxometalate), PMMA (poly(methyl methacrylate)), silicon carbide, tungsten carbide, PFA (perfluoroalkoxy alkane), PP (polypropylene), PE-HD (high-density polyethylene), PE-UHMW (ultra-high-molecular-weight polyethylene), PAS (polyarylsulfone), PA6GF (glass fiber reinforced PA6 (Nylon 6)), PC (polycarbonate), PC GF (polycarbonate with glass fibers), PET (polyethylene terephthalate), PET GX (polyethylene terephthalate glycol), PEEK (polyether ether ketone), PEEK GF (polyether ether ketone with glass fibers),

To increase the reactivity of the starting material mixture compounds or to induce melting of one or more chemical reactants, if need be, the milling process can be carried out upon heating, or the reactive compounds can be pre-heated prior of the start of the milling process.

Depending on the reactivity of the milled materials and the intensity of the milling process, the milling time can vary between 1 minute and 4 hours, for example to 2.5 hours and 3 hours.

According to an advantageous embodiment, the mechanochemical process is a substantially solvent-free process, more precisely in these residual solvents, crystalline solvates, water content as residual water or bound as hydrates comprised in the composition are not meant by the feature "solvent-free", thus meaning that a "substantially solvent-free process" is a process that is substantially free from additional solvent or solvent added during or before the process).

The mechanochemical process that is performed in the invention is performed by enhancing the activity of the reactants with mechanical energy, such as impact and friction, and is performed generally with equipment capable of performing the mechanochemical reaction. Examples of the equipment include one having a reaction vessel and an agitation medium applying mechanical energy, and specific examples thereof include a ball mill, such as a planetary ball mill and a mixer mill, and a mixer, such as shaker. Among these, the use of a planetary ball mill is preferred in view of the agitation efficiency and the energy to be applied.

Reaction vessel can also be understood as being the container.

By way of example and in a non-limiting manner, equipment such as drum mill, vibrating eccentric mill, symoloyer or dynomill can be used.

In the context of the process according to the invention, the hydrogen surrogate may be solid.

According to another embodiment of the process of the invention, the mechanochemical process comprises the steps of :
a) preparing or providing a starting material mixture of at least one reducing agent, at least one solid hydrogen surrogate and at least one compound containing at least one nitro, nitroso, N-hydroxylamino group, and/or
b) mixing, milling and/or grinding said starting material mixture, which includes no metal catalyst, in a reacting environment which includes no metal catalyst, no molecular H₂ and no base, subjected to a mechanical force to generate a mechanochemical reaction between the at least one reducing agent, at least one solid hydrogen surrogate and at least one compound containing at least one nitro, nitroso, N-hydroxylamino group , to obtain an amine derivative,
said mechanical forces occurring in a device chosen among : planetary ball-mill, resonant acoustic mixing (RAM), screw extrusion (single, twin or multi), drum mill, dyno-mill, or vibrating eccentric mill.

In the context of the process according to the invention, the hydrogen surrogate may be solid.

According to a particular embodiment of the process of the invention, the hydrogen surrogate is a solid compound selected in the group of Amberlite^{™} IR-120, boric acid, citric acid, NaHSO₄, NH₄Cl, B₂(OH)₄, NH₄Cl, sodium borohydride.

In the context of the process according to the invention, the hydrogen surrogate and the compound containing at least one nitro, nitroso-, N-hydroxylamino group may be solid.

According to an advantageous embodiment of the process of the invention, the at least one compound containing at least one nitro, nitroso-, N-hydroxylamino group is solid and at least one reducing agent is solid and the at least one hydrogen surrogate is solid.

In the context of the process according to the invention, the hydrogen surrogate may be solid and the compound containing at least one nitro, nitroso-, N-hydroxylamino group may be liquid.

According to an advantageous embodiment of the process of the invention, the at least one compound containing at least one nitro, nitroso-, N-hydroxylamino group is liquid and at least one reducing agent is solid and the at least one hydrogen surrogate is solid.

In the context of the process according to the invention, the hydrogen surrogate, the reducing agent and the compound containing at least one nitro, nitroso-, N-hydroxylamino group may be solid.

According to an advantageous embodiment of the process of the invention, the at least one compound containing at least one nitro, nitroso-, N-hydroxylamino group is solid and at least one reducing agent is solid, the at least one hydrogen surrogate is solid and at least one reducing agent is solid.

In the context of the process according to the invention, the hydrogen surrogate and the reducing agent may be solid.

According to an advantageous embodiment of the process of the invention, the at least one reducing agent are solid and at least one reducing agent is solid and the at least one hydrogen surrogate is solid.

In the context of the process according to the invention, the hydrogen surrogate may be solid.

According to a particular embodiment of the process of the invention, the hydrogen surrogate is a solid compound selected in the group of Amberlite^{™} IR-120, boric acid, citric acid, NaHSO₄, NH₄Cl, B₂(OH)₄, NH₄Cl, sodium borohydride.

In the context of the process according to the invention, the reactant composition may be solvent-free and parameter η is equal to 0.

According to another embodiment of the process of the invention, the subject matter of the invention concerns a reactant composition that is solvent-free and the parameter η is equal to 0.

In the context of the process according to the invention, the hydrogen surrogate may be solid and the reactant composition may comprise a liquid solvent.

According to another embodiment of the process of the invention, the subject matter of the invention concerns a reactant composition that contains a liquid solvent.

In the context of the process according to the invention, the hydrogen surrogate may be solid.

According to an advantageous embodiment of the process of the invention, the parameter η that is greater than 0 and less than 1, more preferably is comprised from about 0.1 to 0.9, more preferably from about 0.3 to 0.7, more preferably from about 0.4 to 0.6 or equal to about 0.5 or about 0.75 µL/mg.

As used herein the value for example "about 0.5" refers to said value which can vary from 5 to 10%.

According to another particular embodiment, the weight ratio of the reducing agent to the hydrogen surrogate is from 1 to 5, preferably from 1 to 3.5, more preferably to 1 to 2.

In the context of the process according to the invention, the hydrogen surrogate may be different from the reducing agent.

According to an advantageous embodiment of the process of the invention, the at least one hydrogen surrogate is different from the at least one reducing agent.

In this embodiment, an additional hydrogen surrogate is present in addition to the reducing agent.

In the context of the process according to the invention, the solid hydrogen surrogate may be different from the reducing agent.

According to an advantageous embodiment of the process of the invention, the at least one hydrogen surrogate is solid and is different from the at least one reducing agent.

In this embodiment, an additional solid hydrogen surrogate is present in addition to the reducing agent.

In the context of the process according to the invention, the hydrogen surrogate may be different from H₂O and from the reducing agent.

According to an advantageous embodiment of the process of the invention,

The at least one hydrogen surrogate is different from H₂O and is different from the at least one reducing agent.

In this embodiment, hydrogen surrogate can be either liquid or solid.

In the context of the process according to the invention, the compound to be reduced may comprise a nitro group.

According to another embodiment of the process of the invention, the mechanochemical process comprises the steps of :
a) preparing or providing a starting material mixture of at least one reducing agent, at least one solid or liquid hydrogen surrogate and at least one compound containing at least one nitro and/or
b) mixing, milling and/or grinding said starting material mixture, which includes no metal catalyst, in a reacting environment subjected to a mechanical force to generate a mechanochemical reaction between the at least one reducing agent, at least one solid or liquid hydrogen surrogate and at least one compound containing at least one nitro, to obtain an amine derivative,
said mechanical force occurring in a a device chosen among : planetary ball-mill, resonant acoustic mixing (RAM), screw extrusion (single, twin or multi), drum mill, dyno-mill, or vibrating eccentric mill.

In the context of the process according to the invention, the compound to be reduced may comprise a nitroso group.

According to another embodiment of the process of the invention, the mechanochemical process comprises the steps of :
a) preparing or providing a starting material mixture of at least one reducing agent, at least one solid or liquid hydrogen surrogate and at least one compound containing at least one nitroso, and/or
b) mixing, milling and/or grinding said starting material mixture, which includes no metal catalyst, in a reacting environment subjected to a mechanical force to generate a mechanochemical reaction between the at least one reducing agent, at least one solid or liquid hydrogen surrogate and at least one compound containing at least nitroso, to obtain an amine derivative,
said mechanical force occurring in a device chosen among: planetary ball-mill, resonant acoustic mixing (RAM), screw extrusion (single, twin or multi), drum mill, dyno-mill, or vibrating eccentric mill.

In the context of the process according to the invention, the compound to be reduced may comprise a N-hydroxylamino group.

According to another embodiment of the process of the invention, the mechanochemical process comprises the steps of :
a) preparing or providing a starting material mixture of at least one reducing agent, at least one solid or liquid hydrogen surrogate and at least one compound containing at least one N-hydroxylamino group, and/or
b) mixing, milling and/or grinding said starting material mixture, which includes no metal catalyst, in a reacting environment subjected to a mechanical force to generate a mechanochemical reaction between the at least one reducing agent, at least one solid or liquid hydrogen surrogate and at least one compound containing at least one N-hydroxylamino group, to obtain an amine derivative,
said mechanical force occurring in a device chosen among : planetary ball-mill, resonant acoustic mixing (RAM), screw extrusion (single, twin or multi), drum mill, dyno-mill, or vibrating eccentric mill.

### DESCRIPTION OF THE FIGURES

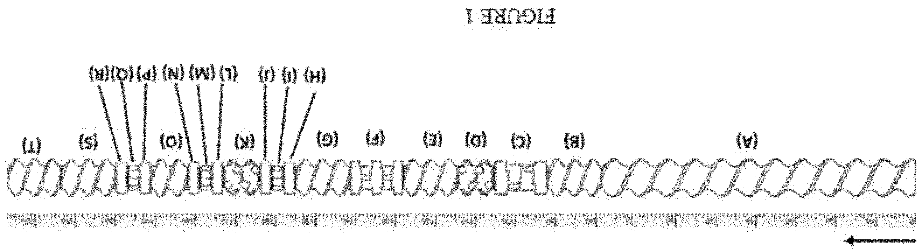

Figure 1 corresponds to the detailed configuration of a ZE9 twin-screw extruder, the numbers displayed are millimeters, as follows:
1. **LD 25:1 twin screw ((A)** ; **OrderNr: C04094):** A primary twin screw element with a length of 78.5 mm and a pitch of 13.5 mm, ensuring efficient conveying of materials.
2. **Concave screw elements ((B), (E), (G), (S) ; OrderNr: C03930):** Multiple concave elements with a length of 13.5 mm and a pitch of 9 mm, placed at various intervals to aid in consistent material transport and initial mixing.
3. **Kneading blocks:**
   ∘ **OrderNr: ((C) ; C03935):** A kneading block with a length of 13.5 mm, 60° phase angle, and 4 kneading segments, positioned to provide initial intensive shear.
   ∘ **OrderNr: ((F) ; C09042):** Another kneading block with a length of 13.5 mm, 90° phase angle, and 5 kneading segments, enhancing the mixing and kneading process.
   ∘ **OrderNr: ((H), (I), (J), (L), (M), (N), (P, (Q), (R) ; C03291 and C03292:** Several shorter kneading elements with lengths of 3 mm, phase angles of 0° and 90°, respectively, strategically placed to continue the shearing and kneading process.
4. **Mixing screw elements:**
   ∘ **OrderNr: ((D); C04871):** A mixing element with a length of 9 mm and a pitch of 9 mm, designed to ensure thorough mixing of the reactants.
   ∘ **OrderNr: ((K); C08930):** An inverted mixing element with a length of 9 mm and a pitch of 9 mm, creating backflow to enhance mixing efficiency.
5. **Additional concave elements ((O) ; OrderNr: C03278 and C03930** : Further concave elements with the same dimensions as previously mentioned, placed to aid in the consistent conveyance and mixing of the materials towards the end of the process.
6. **End screws and segments:**
   ∘ **OrderNr: C03938:** End screw elements with no pitch, ensuring complete discharge of the material from the extruder.
   ∘ **OrderNr: ((T) ; C04872:)** End segment with a concave shape, positioned at the final stage to ensure thorough processing and discharge of the extrudate.

The present invention will be described in more detail with reference to examples below.

### Example 1

### Preparation of o-phenylenediamine by ball milling

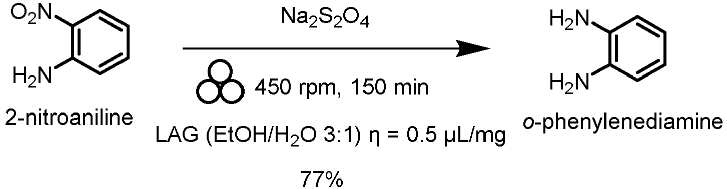

2-nitroaniline (560 mg, 4.05 mmol) and Na₂S₂O₄ (2823.4 mg, 16.22 mmol, 4 eq) were placed in a 45 mL ZrO₂ jar charged with 40x5 mm ZrO₂ balls. Then, LAG additive was added (EtOH, 1269 *µ*L; H₂O, 423 *µ*L; *η* = 0.5 *µ*L/mg) and milled at 450 rpm for 150 min.

To the resulting reaction crude, EtOAc (25 mL) was added. The mixture was filtered through a fritted funnel and the filtrate was collected. The solid residue was washed with EtOAc (3x5 mL). The organic phases were combined and evaporated at reduced pressure to afford o-phenylenediamine as a dark brown solid (338.7 mg, 77% yield).

¹H NMR (400 MHz, DMSO-*d₆*) δ 6.49 (dd, ³J = 5.7 Hz, ⁴J = 3.3 Hz, 2H), 6.37 (dd, ³J = 5.7 Hz, ⁴J = 3.3 Hz, 2H), 4.61 (s, 4H, NH₂).

GC-MS (EI) *m*/*z* calculated for C₆H₈N₂ 108.07, 108 (M·).

Equipment used: Planetary ball-mill (Fritsch Pulverisette 7 premium line of the company Fritsch)

### Example 2

### Preparation of 2-(trifluoromethyl)benzene-1,4-diamine by ball milling

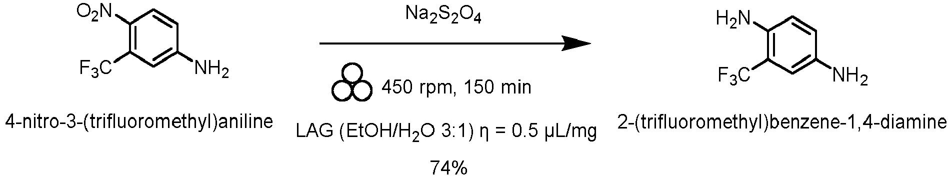

4-nitro-3-(trifluoromethyl)aniline (210 mg, 1.02 mmol) and Na₂S₂O₄ (709.8 mg, 4.08 mmol, 4 eq) were placed in a 12 mL hardened stainless steel jar charged with 20x5 mm hardened stainless steel balls. Then, LAG additive was added (EtOH, 345 *µ*L; H₂O, 115 *µ*L; *η* = 0.5 *µ*L/mg) and milled at 450 rpm for 150 min.

To the resulting reaction crude, EtOAc (15 mL) was added. The mixture was filtered through a fritted funnel and the filtrate was collected. The solid residue was washed with EtOAc (3x5 mL). The organic phases were combined and evaporated at reduced pressure to afford 2-(trifluoromethyl)benzene-1,4-diamine as a dark solid (107.8 mg, 74% yield).

GC-MS (EI) *m*/*z* calculated for C₇H₇F₃N₂ 176.06, 176 (M·).

Equipment used: Planetary ball-mill (Fritsch Pulverisette 7 premium line of the company Fritsch)

### Example 3

### Preparation of 2-chlorobenzene-1,4-diamine by ball milling

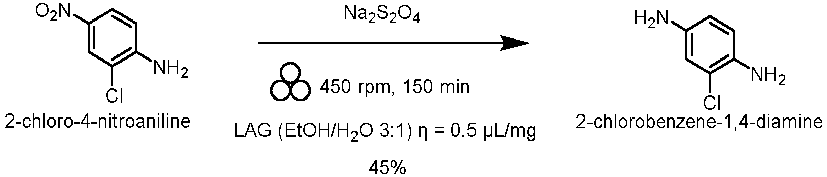

2-chloro-4-nitroaniline (210 mg, 1.02 mmol) and Na₂S₂O₄ (709.8 mg, 4.08 mmol, 4 eq) were placed in a 12 mL hardened stainless steel jar charged with 20x5 mm hardened stainless steel balls. Then, LAG additive was added (EtOH, 345 *µ*L; H₂O, 115 *µ*L; *η* = 0.5 *µ*L/mg) and milled at 450 rpm for 150 min.

To the resulting reaction crude, EtOAc (15 mL) was added. The mixture was filtered through a fritted funnel and the filtrate was collected. The solid residue was washed with EtOAc (3x5 mL). The organic phases were combined and evaporated at reduced pressure to afford a mixture of 2-chloro-4-nitroaniline and 2-chlorobenzene-1,4-diamine (107.8 mg diamine, 45% yield).

GC-MS (EI) *m*/*z* calculated for C₆H₇ClN₂ 143.02, 143 (M·).

Equipment used: Planetary ball-mill (Fritsch Pulverisette 7 premium line of the company Fritsch)

### Example 4

### Preparation of o-phenylenediamine by RAM

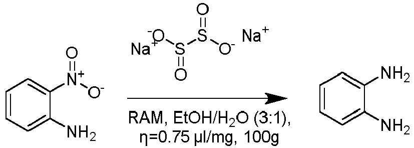

At a 5 mL PTFE jar, where added 700 mg (5.07 mmol, 1 eq) of 2-nitro aniline and 3.53 g of sodium dithionite (20.2 mmol, 4 eq). Then EtOH 2.38ml and water 793 *µ*L were added in a ratio of 3:1 and of a total η value of 0.75 *µ*L/mg. Then the vial was mixed in a LabRAM II Resodyn mixer at 100 *g* for 60 minutes. After the end of the mixing ethyl acetate was added and the mixture was transferred in a filtration funnel and washed further with EtOAc to isolate the insoluble salts from the product. The filtrate was evaporated under reduced pressure and dried over P₂O₅ to afford the desired product as a brown powder (432 mg, 79%).

### NMR:

¹H NMR (400 MHz, DMSO-*d₆*) δ 6.98 - 6.85 (m, 1H), 6.85 - 6.76 (m, 1H).

GC-MS (EI) *m*/*z:* 108 (M·).

Equipment used: LabRAM II Resodyn mixer

### Example 5

### Preparation of 2,4-Diaminophenol by RAM

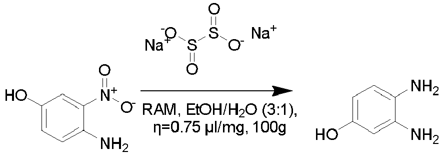

At a 5ml PTFE jar, where added 700mg (4,54 mmol, 1 eq) of 4-amino-3-nitro phenol and 3.16 g of sodium dithionite (18.2 mmol, 4 eq). Then EtOH 2.18ml and water 724 *µ*L were added in a ratio of 3:1 and of a total *η* value of 0.75 *µ*L/mg . Then the vial was mixed in a LabRAM II Resodyn mixer at 100 *g* for 30 minutes. After the end of the mixing ethyl acetate was added and the mixture was transferred in a filtration funnel and washed further with EtOAc to isolate the insoluble salts from the product. The filtrate was evaporated under reduced pressure and dried over P₂O₅ to afford the desired product as a dark powder (304.5mg, 63%).

¹H NMR (400 MHz, DMSO-*d₆*) δ 9.89 (s, 1H), 7.76 (d, *J* = 9.1 Hz, 1H), 7.17 (dd, *J* = 9.1, 2.7 Hz, 1H), 7.10 (d, *J* = 2.3 Hz, 1H), 6.87 (s, 1H), 6.84 (s, 1H), 6.10 (s, 2H), 5.92 (s, 2H).

GC-MS (EI) *m*/*z:* 124 (M·)

Equipment used: LabRAM II Resodyn mixer

### Example 6

### Preparation of o-phenylenediamine by twin screw extrusion:

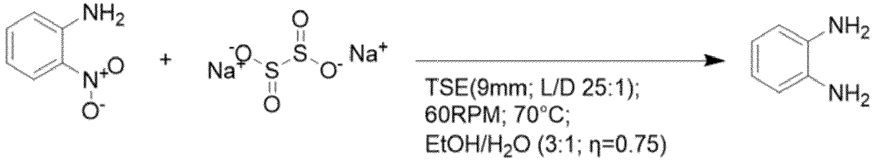

2-nitroaniline (5g, 36.20 mmol) and sodium dithionite (144.80 mmol, 4 eq) are premixed in a mortar and a pestel until the light orange color is homogenous. Then, the premixture is loaded in a gravimetric feeder. A mixture of EtOH/H₂O 3:1 (volumetric ratio) is prepared and charged into a syringe connected to a syringe pump. The barrel of the extruder (ZE9, LD:25:1, Three-Tec) is heated at 70°C. When the barrel is at the target temperature, the solids reactants is introduced into the extruder at 0.5 g/min and simultaneously the LAG is introduced at the second liquid introduction port at 0.375 mL/min (LAG *η* = 0.75 µL/mg). All the extrudate that goes out of the extruder is purge until the extrudate becomes white. The white extrudate is collected in a tube containing 25 mL of ethyl acetate. After the end of extrusion, the mixture of extrudate and ethyl acetate was transferred in a filtration funnel and washed further with EtOAc to isolate the insoluble salts from the product. The filtrate was evaporated under reduced pressure and dried over P₂O₅ to afford the desired product as a brown powder.

The screw configuration used in the ZE9 twin-screw extruder is specifically designed to optimize the mixing and reaction of the components This configuration includes a series of precise screw elements arranged to achieve effective conveying, mixing, and kneading of the materials. (see Figure 1).

The total length of the screw configuration is 227 mm, designed to fit the ZE9 extruder with a length-to-diameter (L/D) ratio of 25:1. This precise arrangement of screw elements is crucial for the effective reduction of 2-nitroaniline in the extrusion process, providing the necessary mixing, shear, and conveyance to achieve the desired reaction outcome.

## Claims

1. Use of a mechanochemical method for selectively reducing a compound containing at least one nitro, nitroso-, N-hydroxylamino group in liquid-assisted grinding (LAG) conditions into an amine, with a metal-free reactant composition comprising at least one reducing agent and at least one liquid hydrogen surrogate, the reactant composition being free from molecular H₂ and being free from base,
said liquid-assisted grinding (LAG) conditions occurring in a device chosen among :
planetary ball-mill, resonant acoustic mixing (RAM), screw extrusion (single, twin or multi), drum mill, dyno-mill, or vibrating eccentric mill.

2. The use according to claim 1, wherein said mechanical method is scalable and said amine is liable to be produced from about 0.1 g to about 1 kg.

3. The use according to any one of claim 1 or 2, in which the reactant composition comprises optionally a further additive, and in which the at least one compound containing at least one nitro, nitroso-, N-hydroxylamino group, and at least one reducing agent are solid.

4. The use according to claim 1 to 3, in which, the liquid hydrogen surrogate is preferably water, an alcohol, and/or a polyol, their mixture thereof, preferably a mixture of EtOH:H₂O.

5. The use according to claim 4, in which, the volumetric ratio of the mixture of EtOH:H₂O is from 5:1 to 1:5, preferably from 3:1 to 1:1, more preferably is 3:1.

6. The use according to any one of claims 1 to 5, in which the LAG conditions are defined by the parameter η that is greater than 0 and less than 1, more preferably is comprised from about 0.1 to 0.9, more preferably from about 0.3 to 0.7, more preferably from about 0.4 to 0.6 or equal to about 0.5 or about 0.75 µL/mg.

7. The use according to any one of claims 1 to 6, in which the at least one reducing agent is sodium dithionite.

8. The use according to any one of claims 1 to 7, in which the compound containing at least one nitro, nitroso-, N-hydroxylamino group is a substituted or unsubstituted nitroaromatic, nitrosoaromatic and/or aromatic hydroxylamine of general formula Ar-Y, wherein Y is NO₂, NO or NHOH and Ar is an aromatic ring with or without one or more substitutions.

9. A mechanochemical process for selectively reducing a compound to be reduced to an amine derivative by reacting the said compound to be reduced with at least one reducing agent and at least one liquid hydrogen surrogate in liquid-assisted grinding (LAG) conditions and in the absence of a catalyst metal, in the absence of molecular H₂ and in the absence of a base,
said liquid-assisted grinding (LAG) conditions occurring in a device chosen among:
planetary ball-mill, resonant acoustic mixing (RAM), screw extrusion (single, twin or multi), drum mill, dyno-mill, or vibrating eccentric mill.

10. The mechanochemical process according to claim 9, which comprises the steps of :
a) preparing or providing a starting material mixture of at least one reducing agent, at least one liquid hydrogen surrogate and at least one compound containing at least one nitro, nitroso, N-hydroxylamino group, and/or
b) mixing, milling and/or grinding said starting material mixture, which includes no metal catalyst, in a reacting environment subjected to a mechanical force to generate a mechanochemical reaction between the at least one reducing agent, at least one liquid hydrogen surrogate and at least one compound containing at least one nitro, nitroso, N-hydroxylamino group, to obtain an amine derivative, and in which the LAG conditions are defined by the parameter η that is greater than 0 and less than 1, more preferably is comprised from about 0.1 to 0.9, more preferably from about 0.3 to 0.7, more preferably from about 0.4 to 0.6 or equal to about 0.5 or about 0.75 µL/mg,

11. The mechanochemical process according to any one of claim 9 or 10, wherein said process is scalable and said amine is liable to be produced from about 0.1 g to about 1 kg.

12. The mechanochemical process according to any one of claim 9 or 11, in which the said mixture comprises optionally a further additive,

13. The mechanochemical process according to any one of claims 9 to 12, in which the liquid hydrogen surrogate is preferably water, an alcohol, and/or a polyol, their mixture thereof, preferably a mixture of EtOH:H₂O,
or,
in which the liquid hydrogen surrogate is a mixture of EtOH:H₂O and the weight ratio of said mixture of EtOH:H₂O is from 5:1 to 1:5, preferably from 3:1 to 1:1, more preferably is 3:1.

14. The mechanochemical process according to any one of claims 9 to 13, in which the at least one reducing agent is sodium dithionite.

15. The mechanochemical process according to any one of claims 9 to 14, in which the compound containing at least one nitro, nitroso-, N-hydroxylamino group is a substituted or unsubstituted nitroaromatic, nitrosoaromatic and/or aromatic hydroxylamine of general formula Ar-Y, wherein Y is NO₂, NO or NHOH and Ar is an aromatic ring with or without one or more substitutions.
